# EUROPEAN PATENT SPECIFICATION

(11) **EP 2 465 062 B1**
(45) Date of publication and mention of the grant of the patent: **06.12.2017**
(21) Application number: 10750034.0
(22) Date of filing: 07.08.2010
(51) Int. Cl.: G06F 19/00, A61B 3/16

(54) **GLAUCOMA COMBINATORIAL ANALYSIS**
KOMBINATORISCHE GLAUKOMUNTERSUCHUNG
ANALYSE COMBINATOIRE APPLIQUÉE AU GLAUCOME

(30) Priority: 10.08.2009 US 232726 P
(43) Date of publication of application: 20.06.2012
(73) Proprietor: Carl Zeiss Meditec AG, 07745 Jena (DE)
(72) Inventor: ZHOU, Qienyuan, Del Mar CA 92014 (US); EVERETT, Matthew, J., Livermore CA 94550 (US); DURBIN, Mary, San Francisco CA 94121 (US)
(74) Representative: Beck, Bernard
(86) International application number: PCT/EP2010/004846
(87) International publication number: WO 2011/018193

(56) References cited:
- EP-A2- 1 666 009
- US-A- 5 878 746
- US-A1- 2006 084 856
- LIN ET AL: "Optic Nerve Head and Retinal Nerve Fiber Layer Analysis" OPHTHALMOLOGY, J. B. LIPPINCOTT CO., PHILADELPHIA, PA, US, vol. 114, no. 10, 28 September 2007 (2007-09-28), pages 1937-1949, XP022277177 ISSN: 0161-6420 DOI: DOI:10.1016/J.OPHTHA.2007.07.005 cited in the application
- AGARWAL HC ET AL: "Target Intraocular Pressure in Glaucoma Managment" ASIAN JOURNAL OF OPHTHALMOLOGY,, vol. 3, no. 3,4, 1 January 2001 (2001-01-01), pages 15-18, XP009142912

## Description

### TECHNICAL FIELD

The subject invention relates to combinatorial analyses of data from two or more diagnostic tests for the detection of eye diseases, simplified interpretation of test results, and assessment of disease stage and rate of change. Of particular interest is the development of combinatorial analyses to improve glaucoma detection and progression rate assessment based on combinations of structural and functional tests. More specifically, approaches are described where data of one or more tests and their normative databases are converted to the distribution and scale of another test for further analysis to detect glaucomatous damage. Approaches are also described where data from more than one test is used to assess stage index and rate of change. In addition, methods for displaying the combinatorial analysis results are disclosed.

### BACKGROUND

Glaucoma is a complex group of neurodegenerative diseases that arises from progressive damage to the optic nerve (ON) and retinal ganglion cells (RGCs) and their axons, the retinal nerve fiber layer (RNFL). Functional measurements of visual sensitivities made with the Humphrey® Field Analyzer and Matrix™ perimeter, structural measurements of the RNFL with optical coherence tomography (OCT) and the GDx™ scanning laser polarimeter, and ONH topographic measurements with the Heidelberg Retina Tomograph (HRT) and OCT are all surrogate measures of the underlying RGC populations. While there is significant correlation between these tests, it is not uncommon for a glaucoma patient to be identified in one test but not in another, and similarly, for a normal subject to be flagged as positive in one test but not in another. The apparent disagreement between tests may be due to test-retest variability, dynamic range difference, confounding factors affecting different tests differently, and quality of the tests.

Clinical studies suggest that these diagnostic tests, used in isolation, provide useful information on the diagnosis and progression of the disease and, used in conjunction, provide supportive and complementing information which could lead to improved accuracy in disease detection and monitoring of progression. However, there is not one single diagnostic test, used in isolation, that provides sufficient diagnostic accuracy and applicability across patient population and disease dynamic range. Multi-modality testing is desired to improve applicability and accuracy. In practice, clinicians are often expected to correlate results from different tests to make a clinical assessment regarding diagnosis and/or progression, usually, based on eyeballing multiple reports. Such a task is difficult and subjective, and highly variable across observers. Combinatorial analysis is a process or method that takes two or more tests, analyzes them separately and in combination, and outputs a result that is simpler and/or more accurate than the full analysis outputs of the original tests. The clinician then makes the clinical assessment as to diagnosis and/or progression based on the simplified output of the combinatorial analysis. Combinatorial analysis is necessary to simplify the interpretation process, ensure consistent and reliable assessment, and improve clinical assessment accuracy, leading to better and quicker clinical decisions.

The subject disclosure is directed to a number of improvements in data analysis algorithms, integration of the analyses, and display techniques for combined glaucoma detection, stage index calculation and rate of change over time, and reporting. These improvements can be implemented using any combination of spatial measurements of structures within the eye and/or functions of the eye that can then be analyzed in accordance with the subject invention for detection and monitoring of eye diseases. US 2006/084856 discloses a combination ophthalmic instrument comprising a non-contact measurement system and a contact measurement system for measuring parameters of the eye, the parameters are being the Intraocular Pressure of the eye. Measurement values from both systems are presented on a display of the instrument. A measurement value generated by one of the systems is automatically adjusted based on a measurement value generated by the other system in accordance with stored correction information.

### SUMMARY

The present invention is defined by the claims and nothing in this section should be taken as a limitation on those claims. Advantageously, embodiments of the present invention overcome the above-described problems in the art and provide analysis techniques and displays improving diagnostic accuracy and consistency.

In one aspect of the subject invention, measurements from individual diagnostic tests are transformed using one or more conversion functions such that the resulting distribution from the various tests are similar to each other to facilitate qualitative and quantitative comparison. The conversion maximizes the similarity of the results of the different tests across the patient population. Available normative databases of different modalities are converted to the common distribution and scale to facilitate the analysis.

In another aspect of the subject invention, the degree of abnormality in a patient's eye is analyzed using measurements from two or more diagnostic tests. A function that is optimized to discriminate between normal and diseased is applied to the two measurements and the resulting output is compared to a probability distribution created from measurements on normal eyes. The state of the function relative to normal is displayed. A further aspect of the invention is also displaying the functional output. The functional output may be in the same form as one of the inputs and the inputs to the function can be weighted according to the reliability of the individual diagnostic tests.

In another aspect of the subject invention, the combinatorial analyses are parameterized into global, regional and local measures for a multi-modal measurement confirmation because glaucoma damage has different morphological appearances.

In another aspect of the subject invention, the combinatorial analyses are simplified for more objective interpretation of test results through data reduction because current interpretation of multi-modality data is subjective and lacks consistency. Such data reduction methods include machine learning classification, machine learning regression, and combination of probabilities.

In another aspect of the subject invention, the progression of disease in a patient's eye is analyzed using measurements from two or more diagnostic tests to create a function that generates an output that measures the stage of disease and comparing the output of the function at subsequent patient visits. This can be accomplished by calculating a stage index for individual modalities and presented in a common scale. In another aspect of the subject invention, a combined stage index is calculated to improve stage assessment accuracy and dynamic range coverage. In another aspect of the subject invention, stage indices can be a global index or a plurality of regional indices. In another aspect of the subject invention, stage index may be generated from combining stage indices of different modalities or from the combined measurement by combining measurements of different modalities. In another aspect of the invention, the measurements can be compared to a probability distribution of the repeatability of the functional output generated from normal subjects to indicate a likelihood of disease progression.

In another aspect of the subject invention, display techniques were developed to provide overall interpretation for disease detection and detailed assessment of damage. The display technique involves displaying multiple output parameters from different diagnostic tests as a function of time on a single graphical display. The overall interpretation includes a classifier and an agreement index ("AI") as further aspects of the invention. In another aspect of the invention, the display provides detailed assessment of global, regional and local damage. In another aspect of the invention, clinically useful information that impacts disease was also displayed, including trend assessment, treatment data, and treatment information. The trend assessment can be generated from the combined measurement or from the measurement of the individual modalities.

In all aspects of the invention the diagnostic tests can include combinations of structural and functional diagnostic tests including visual field testing, RNFL analysis, ONH analysis, ganglion cell analysis and macular inner retinal thickness. The diagnostic tests can be performed using perimetry, scanning laser polarimetry, and optical coherence tomography (OCT). Multiple diagnostic tests can be performed using the same technology.

The combined analysis of test results from different modalities is very important in detecting and monitoring disease. The combined analysis of RGC and its surrogates is very important in detecting and monitoring glaucomatous disease. A reliable combinatorial analysis method and a comprehensive and easy-to-understand report are therefore extremely desirable, for both the clinicians and the patients. The subject invention meets a long-felt and unsolved clinical need.

### BRIEF DESCRIPTION OF THE DRAWINGS

FIG. 1a shows the output of a visual field test of a patient known to have glaucoma but the test results indicate that the patient falls within normal limits. FIG 1b shows the same patient's GDx output indicating substantial diffuse RNFL loss in the right eye (OD) supporting the glaucoma diagnosis.
FIG. 2 shows a map that relates the regions of a 24-2 HFA field to the optic disc sectors
FIG. 3 shows a diagram illustrating the idea of 3 modality combinatorial analysis and key elements in one exemplary embodiment.
FIG. 4 shows a diagram illustrating the challenge of conversion between RNFL measurements and visual field measurements posed by inter-subject variation of the RNFL pattern. All 3 RNFL images are from normal eyes.
FIG. 5 shows a diagram that illustrates an approach for the combined glaucoma detection.
FIG. 6 shows a diagram that illustrates an alternative approach for the combined glaucoma detection.
FIG. 7 shows a diagram that illustrates localized combined analysis and display for glaucoma detection.
FIG. 8 shows a diagram that illustrates variation to localized combined analysis and display for glaucoma detection.
FIG. 9 shows a diagram that illustrates regional combined analysis based on GHT zones.
FIGS. 10a and 10b shows a diagram that illustrates the steps and alternatives for the development of machine learning classifier (MLC).
FIG. 11 shows a diagram that illustrates the steps for stage index assessment, rate of change assessment, and progression event detection.
FIG. 12 shows a diagram that illustrates the alternative stage index calculation based on VFI calculation in HFA.
FIG. 13 shows a diagram illustrating the opportunities in data display.

### DETAILED DESCRIPTION

### Glaucoma Testing

In clinical practice, the presence of one or more glaucoma risk factors (such as elevated IOP, family history, disc hemorrhage, etc.) or signs of glaucoma from clinical examination (such as the appearance of the optic disc), leads to further testing that may include testing of the visual field (VF), and evaluation of the optic nerve (ON) and the retinal nerve fiber layer (RNFL) beyond clinical examination by ophthalmoscopy. Abnormality consistent with a glaucomatous damage pattern found in clinical examination and these tests is the basis for making a diagnosis.

Following the diagnosis, a clinician may decide to initiate treatment to lower IOP and monitor treatment response if the patient's risk for imminent consequential further damage is high or monitor patient for signs of progression without initiating treatment if the patient's risk for imminent consequential further damage is low. A patient's risk for further damage depends on: 1) age, IOP, disc hemorrhage, etc., 2) severity of damage (i.e. disease stage) when the glaucoma is first discovered, and 3) the rate of change (i.e. progression of disease stage) if the patient has been followed over a period of time.

From the glaucoma testing, the clinician tries to assess the following:
1) Does the patient have glaucoma (i.e. detection)?
2) How severe is the patient's glaucoma damage (i.e. disease stage)?
3) Is the patient getting worse (i.e. progression event detection)?
4) Is the patient getting worse so fast as to risk vision impairment (i.e. rate of change)?

Individual test modalities, such as the Humphrey® Field Analyzer (HFA), Matrix™ perimeter, Stratus OCT™ retinal imager, Cirrus™ HD-OCT, GDx™ scanning laser polarimeter, and Heidelberg Retina Tomograph (HRT), all strive to provide information to help clinicians answer these questions. However, as discussed in the next section, to date, there is not one single clinical device that, used in isolation, satisfies the clinical needs in glaucoma testing across the patient population and across the disease dynamic range. In practice, clinicians are often expected to subjectively correlate results of at least a couple of glaucoma tests to make a diagnosis. Subjective interpretation of test results is time-consuming and lacks consistency across observers.

The purpose of combinatorial analysis for multi-modality testing is to simplify the interpretation process, improve diagnostic accuracy and disease stage assessment, and improve workflow and quality of care by combining tests of two or more individual test modalities.

### The Need for Multi-Modality Testing

Functional measurements of visual sensitivities and structural measurements of RNFL thickness and optic nerve head topography are all dependent, in part, on the underlying populations of RGCs. These measurements are used to detect glaucoma and to monitor disease progression in glaucoma management, as a reflection of the pathological loss of RGCs and their axons. It has been demonstrated that a reduction of visual sensitivity in an area of the visual field is proportional to the amount of loss of RGCs in the corresponding area of the retina (RS Harwerth et al. "Visual field defects and retinal ganglion cell losses in patients with glaucoma" Arch Ophthalmol (2006) 124:853-859 and HA Quigley et al. "Retinal ganglion cell atrophy correlated with automated perimetry in human eyes with glaucoma" Am J Ophthalmol (1989) 107:453-464) and, proportional to the loss of RGC axons entering the optic nerve from the same retina area. Consequently, it would be expected that visual sensitivity measurements and RNFL thickness/ONH topography measurements are highly correlated measures of the underlying populations of RGCs. This expectation of correlated structure-function relationships in glaucoma has been confirmed for the progressive effects of experimental glaucoma in monkeys (RS Harwerth et al. "The relationship between nerve fiber layer and perimetry measurements" Invest Ophthalmol Vis Sci (2007) 48:763-773) and cross-sectional studies of glaucoma patients with varying stages of the disease (DF Garway-Heath et al. "Mapping the visual field to the optic disc in normal tension glaucoma eyes" Ophthalmology (2000) 127:674-680, TA Beltagi et al. "Retinal nerve fiber layer thickness measured with optical coherence tomography is related to visual function in glaucomatous eyes" Ophthalmology (2003) 110:2185-2191, NJ Reus et al. "The relationship between standard automated perimetry and GDx VCC measurements" Invest Ophthalmol Vis Sci (2004) 45:840-845 and LA Kerrigan-Baumrind et al. "Number of Ganglion Cells in Glaucoma Eyes Compared with Threshold Visual Field Tests in the Same Persons" Invest Ophthalmol Vis Sci (2000) 41:741-748).

Inter-subject variability and test-retest variability present serious challenges to early detection of glaucoma. For instance, it has been observed that ganglion cell losses of 40% to 50% were necessary before visual sensitivity losses exceeded the normal 95% confidence limits [HA Quigley et al. "Optic Nerve Damage in Human Glaucoma. III. Quantitative correlation of Nerve Fiber Loss and Visual Field Defect in Glaucoma, ischemic Optic Neuropathy, Papilledema, and Toxic Neuropathy" Arch Ophthalmol (1982) 100:135-146 and HA Quigley et al. "Retinal Ganglion Cell Atrophy Correlated with Automated Perimetry in Human Eyes with Glaucoma" AM J Ophthalmol (1989) 107:453-464) and RNFL losses of approximately 30% were necessary before GDx measurements exceeded the normal 95% confidence limits (based on GDx normative limits for TSNIT Average). To date, none of the glaucoma tests, used in isolation, has achieved satisfactory accuracy required for glaucoma diagnosis or for glaucoma progression detection (LK Singh et al. "Optic Nerve Head and Retinal Nerve Fiber Layer Analysis - A Report by the American Academy of Ophthalmology" Ophthalmology (2007) 114:1937-1949 and MF Delgado et al. "Automated perimetry: a report by the American Academy of Ophthalmology" Ophthalmology (2002) 109:2362-2374).

Since HFA, GDx, OCT, and HRT provide surrogate measures of retinal ganglion cells based on different traits, it is not surprising that these tests may differ in a patient's eye in:
Sensitivity to detecting existing damage
Sensitivity to detecting ongoing damage (i.e. progression)
Confounding factors/artifacts
Measurement variability (precision)

Medeiros et al. compared GDx VCC, HRT II, and Stratus OCT for discrimination between healthy eyes and eyes with glaucomatous visual field loss (FA Medeiros et al. "Comparison of the GDx VCC Scanning Laser Polarimeter, HRT II Confocal Scanning Laser Ophthalmoscope, and Stratus OCT Optical Coherence Tomograph for the Detection of Glaucoma" Arch Ophthalmol (2004) 122:827-837). The study included 107 patients with glaucomatous visual field loss and 76 healthy subjects of a similar age. After the exclusion of subjects with unacceptable measurements with reliability failure, the final study sample included 141 eyes of 141 subjects (75 with glaucoma and 66 healthy control subjects). This means 30% of glaucoma subjects and 13% of normal subjects could not be evaluated by one or more of the 3 tests. However, of the total 42 subjects with reliability failures, only two (2) subjects (1%) could not be evaluated by all 3 tests. Therefore, better patient coverage or applicability can be achieved with access to more than one test modality. While this study only compares structural devices, similar complementary applicability can be expected between structural tests and functional tests. In this study population, Mean ± SD of the visual field MD parameter for patients with glaucoma was -4.87 ± 3.9 dB, and 70% of these patients had early glaucomatous visual field damage. No statistically significant difference was found between the areas under the receiver operating characteristic curves (AUROCs) for the best parameters from the 3 modalities. On average, at specificity of 95%, the sensitivity is approximately 62% based on any single structural testing. This means approximately 38% of glaucoma patients with visual field loss will not be detected with any single structural test in this study population.

The above study illustrates the limitation on structural testing alone; similar limitation exists with isolated functional testing. Reus et al. reported thinning of the RNFL detected with GDx VCC in perimetrically unaffected eyes of glaucoma patients with field loss in their fellow eyes. The NFI had a value of ≥40 in 11 of the 23 (47.8%) perimetrically unaffected eyes of the glaucoma patients, 19 of 23 (82.6%) eyes with VF loss of the glaucoma patients, and 3 of 73 (4.1%) of the healthy control eyes (NJ Reus et al. "Scanning Laser Polarimetry of the Retinal Nerve Fiber Layer in Perimetrically Unaffected Eyes of Glaucoma Patients Ophthalmology" (2004) 111:2199-2203).

Agreement between different tests is usually moderate, when comparing structural tests, functional tests, or structural and functional tests. For example, chance-corrected agreement was 0.72 between GDx and Stratus, 0.50 between GDx and HRT, and 0.55 between Stratus and HRT (FA Medeiros et al. "Comparison of the GDx VCC Scanning Laser Polarimeter, HRT II Confocal Scanning Laser Ophthalmoscope, and Stratus OCT Optical Coherence Tomograph for the Detection of Glaucoma" Arch Ophthalmol (2004) 122:827-837). It is to be noted that the agreement in detecting the presence of disease between tests will vary based on disease stage; better agreement is expected in patients with advanced damage and poorer agreement in patients with early damage. Therefore, the benefits of multi-modality testing and combinatorial analysis are likely to be most appreciable for early disease detection.

Similar observations regarding agreement between tests have been reported in glaucoma progression detection. Chauhan et al. investigated the relationship between optic disc changes measured with HRT and those measured with HFA in a study population of 77 patients with early glaucomatous visual field damage followed for a median of 5.5 years (BC Chauhan et al. "Optic Disc and Visual Field Changes in a Prospective Longitudinal Study of Patients With Glaucoma - Comparison of Scanning Laser Tomography With Conventional Perimetry and Optic Disc Photography" Arch Ophthalmol (2001)119:1492-1499). Twenty-one (21) patients (27%) showed no progression with either technique. Thirty-one (31) patients (40%) progressed with HRT only, while 3 (4%) progressed with HFA only, and 22 patients (29%) progressed with both techniques. In a more recent longitudinal study, Artes and Chauhan reported that current progression detection based on HFA and HRT provide largely independent measures of progression (P. Artes et al. "Longitudinal changes in the visual field and optic disc in glaucoma" Progress in Retinal and Eye Research (2005) 24:333-354).

The benefits of multi-modality testing and combinatorial analysis are not limited to structure-function combinations only. For example, OCT (Stratus and Cirrus) and GDx both measure the RNFL structure, but based on different traits of the tissue. OCT measures RNFL thickness (T) and GDx measures RNFL retardation (R) which is proportional to RNFL thickness (T) and birefringence (Δn): R = (Δn)* T. RNFL birefringence varies with position around the ONH, being higher in superior and inferior regions, and lower in temporal and nasal regions (X-R Huang et al. " Microtubules Contribute to the Birefringence of the Retinal Nerve Fiber Layer" Invest Ophthalmol Vis Sci (2005) 46:4588-4593). Birefringence depends on RNFL ultrastructure, which may change before RNFL thickness in early glaucoma, as suggested in recent studies (B Fortune et al. "Retinal Nerve Fiber Layer Birefringence Declines Prior to Thickness After Onset of Experimental Glaucoma or Optic Nerve Transection in Non-Human Primates" Invest Ophthalmol Vis Sci (Suppl) (2008) 49: abstract #3761 and E. Gotzinger et al. "Retinal Nerve Fiber Layer Birefringence of Healthy and Glaucomatous Eyes Measured with Polarization Sensitive Spectral Domain OCT" Invest Ophthalmol Vis Sci (Suppl) (2008) 49: abstract #3762). The possibility of early detection based on changes in the ultrastructure could potentially open up a window of opportunity for glaucoma treatment before axonal loss. Birefringence change can be differentiated from thickness change by combining Cirrus and GDx measurements. In addition, glaucomatous damage to the papillo-macular bundles can be monitored with OCT (correlation with visual field: r = 0.75) but not with GDx (no correlation with visual field) due to low birefringence in temporal region, illustrating another benefit of combining the tests (FK Horn et al. "Correlation Between Local Glaucomatous Visual Field Defects and Loss of the Nerve Fiber Layer Thickness Measured with Polarimetry (GDx) and Spectral Domain OCT" Invest Ophthalmol Vis Sci (Suppl) (2008) 49: abstract #732).

Any two (2) glaucoma tests, regardless of the specific technology (structural or functional), could complement each other when each is sensitive to change during a different stage in the disease progression, or if they differ in applicability to certain populations or to certain stages of disease. Performance gain is expected to be greater when combining tests with less overlap so combining structural and functional tests fall into this category.

### The Need to Simplify Interpretation

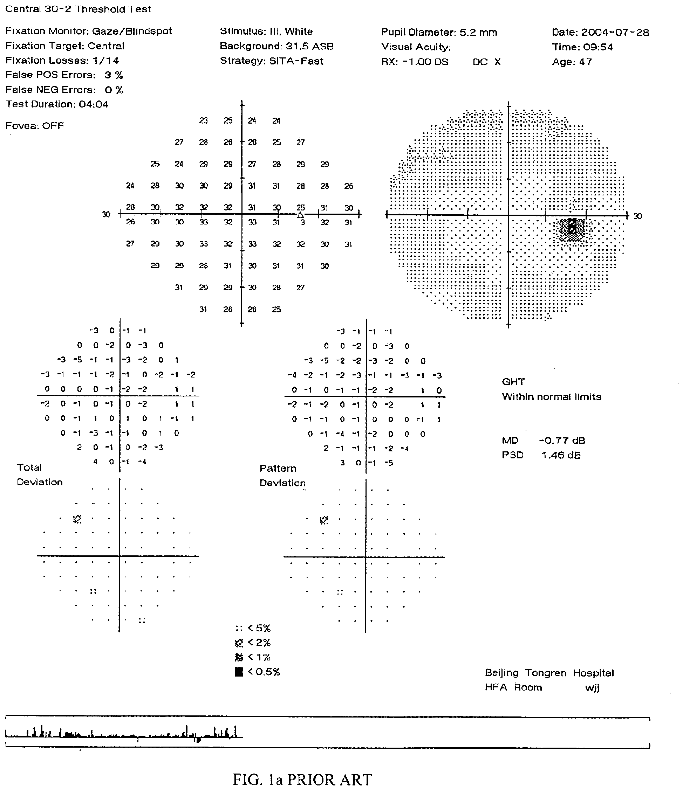

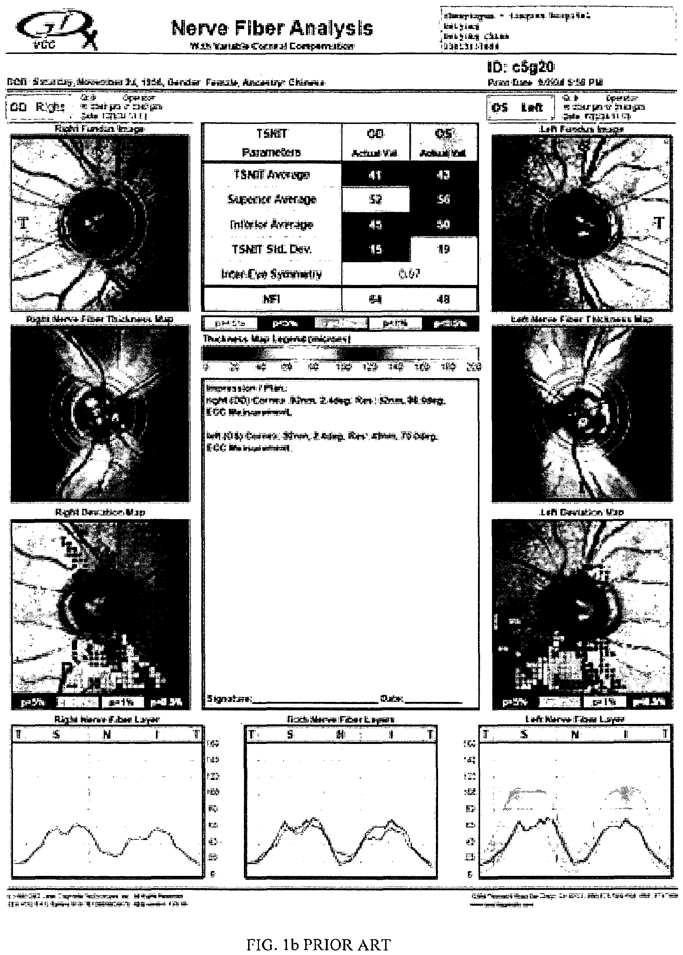

In clinical practice, clinicians are expected to review test reports by HFA, OCT, or GDx, and make their interpretation, which is time consuming and, lacks consistency across observers. A glaucoma subject's visual field test results with HFA (right eye shown) is shown in Figure 1a while the same patient's RNFL test results with GDx (both eyes shown) is shown in FIG. 1b to illustrate the complexity of interpretation.

For each modality, clinicians are required to review multiple aspects of a report. For example, while interpreting a single HFA test report, a clinician must review test reliability data, rule out measurement artifacts (droopy lids, cataract, correction lens artifacts, and learning effects, etc.), and then make diagnostic assessment following, for example, a set of guidelines for number of parameters including Glaucoma Hemifield Test (GHT), Corrected Pattern Standard Deviation (CPSD), and pattern deviation plot (DR Anderson Automated Static Perimetry St. Louis: Mosby-Year Book 1992). Similarly, interpreting a single GDx RNFL test report requires a clinician to review image quality information, rule out measurement artifacts (such as atypical scans, saturated area caused by peripapillary atrophy, etc.), and then make a diagnostic assessment based on reviewing a number of global and local parameters including summary parameters (temporal-superior-nasal-inferior-temporal (TSNIT) average, Superior average, Inferior average, etc.), machine learning classifier (NFI) result, RNFL TSNIT plot, and RNFL image deviation map.

The interpretation of multi-modality data creates additional challenges. The GDx test is centered on the optic nerve head (ONH) and the visual field test is centered on the fovea. Correlating test locations between different test modalities poses one level of challenge. The increased data dimension poses another level of challenge. For example, the right eye of the subject in FIG. 1 tested normal with HFA (FIG. 1a) but exhibits diffuse RNFL damage with GDx (FIG. 1b). It is not apparent what the overall assessment should be. In the absence of algorithms to combine multi-dimensional data, the overall assessment for disease diagnosis will vary from observer to observer.

### Correlating Structural and Functional Tests

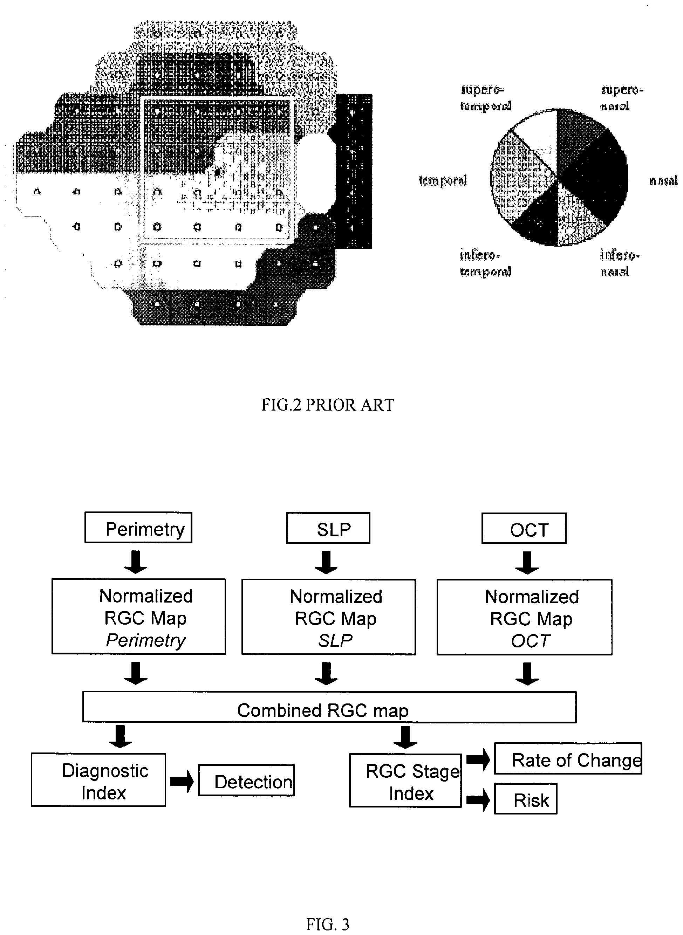

Correlating regions of visual field with sectors of the optic disc is often based on the map developed by Garway-Heath et al. (DF Garway-Heath et al. "Mapping the visual field to the optic disc in normal tension glaucoma eyes" Ophthalmology (2000) 127:674-680). As shown in FIG 2, the 52 visual field test locations are grouped into 6 regions, corresponding to 6 sectors in the optic disc. Several studies correlating visual field results with HRT, OCT, and/or GDx measurements employed this map (TA Beltagi et al. "Retinal nerve fiber layer thickness measured with optical coherence tomography is related to visual function in glaucomatous eyes" Ophthalmology (2003) 110:2185-2191, NJ Reus et al. "The relationship between standard automated perimetry and GDx VCC measurements" Invest Ophthalmol Vis Sci (2004) 45:840-845, E. Gotzinger et al. "Retinal Nerve Fiber Layer Birefringence of Healthy and Glaucomatous Eyes Measured with Polarization Sensitive Spectral Domain OCT" Invest Ophthalmol Vis Sci (Suppl) (2008) 49: abstract #3762, and FK Horn et al. "Correlation Between Local Glaucomatous Visual Field Defects and Loss of the Nerve Fiber Layer Thickness Measured with Polarimetry (GDx) and Spectral Domain OCT" Invest Ophthalmol Vis Sci (Suppl) (2008) 49: abstract #732). Correlation coefficients (r) up to 0.75-0.80 were reported for Superior-Temporal and Inferior-Temporal sectors (FK Horn et al. "Correlation Between Local Glaucomatous Visual Field Defects and Loss of the Nerve Fiber Layer Thickness Measured with Polarimetry (GDx) and Spectral Domain OCT" Invest Ophthalmol Vis Sci (Suppl) (2008) 49: abstract #732).

More recently, a point-wise conversion model from a GDx VCC RNFL image to a visual field sensitivity map was reported by Zhu et al. (H Zhu et al. "Combining Structural and Functional Measurements to Improve Reproducibility of Follow Up Data in Glaucoma" Invest Ophthalmol Vis Sci (2009) Abs #2572). The model was developed using a Bayesian Radial Basis Function from a set of clinical data for the purpose of reducing variability in glaucoma follow-up by generating a combined visual field through the weighted mean of the converted visual field sensitivity map and the measured visual field sensitivity map.

Harwerth et al. developed a model to predict the ganglion cell density underlying a given level of visual sensitivity and location in the visual field based on an experimental glaucoma model and have applied the model to clinical perimetry successfully (RS Harwerth et al. "Visual field defects and retinal ganglion cell losses in patients with glaucoma" Arch Ophthalmol (2006) 124:853-859 and RS Harwerth et al. "Neural Losses Correlated with Visual Losses in Clinical Perimetry" Invest Ophthalmol Vis Sci (2004) 45:3152-3160). The model assumes linear structure-function relationships on log-log coordinates, with slope and intercept parameters varying systematically with eccentricity. In another application of the model (RS Harwerth et al. "The relationship between nerve fiber layer and perimetry measurements" Invest Ophthalmol Vis Sci (2007) 48:763-773), the number of ganglion cells derived from SAP and OCT data for normal eyes and experimental glaucoma eyes were in close agreement on average, however, large individual variation was observed.

Hood et al. also proposed a simple linear model to relate a lower region and an upper region of SAP field data to the superior-temporal sector and inferior-temporal sector of OCT data (DC Hood et al. "A Framework for Comparing Structural and Functional Measures of Glaucoma Damage" Progress in Retinal and Eye Research (2007) 26:688-710). Their model assumes that the RNFL thickness measured with OCT has two components, one component is the axons of the retinal ganglion cells and the other, the residual, is glial cells and blood vessels, etc. The axon portion is assumed to decrease in a linear fashion with losses in SAP sensitivity (in linear units); the residual portion is assumed to remain constant.

The work published by Swanson et al. describes another alternative model to correlate perimetric defects with the loss of ganglion cell numbers taking into account eccentricity and glaucoma damage (WH Swanson et al. "Perimetric Defects and Ganglion Cell Damage: Interpreting Linear Relations Using a Two-Stage Neural Model" Invest Ophthalmol Vis Sci (2004) 45:466-472).

These publications address some aspects of combining structural and functional tests, but none provides an integrated solution to address the clinical needs for multi-modality testing and combinatorial analysis. An integrated solution simplifies presentation of results, increases confidence in the reported outcome, and improves diagnostic efficacy or sensitivity to change.

### Overview

Detection of glaucoma immediately impacts a clinician's decision on patient management. Similarly, knowing the stage of the disease helps a clinician assess the risk of imminent consequential further damage, which also directly impacts the clinical decision. Further, knowing an individual patient's rate of progression allows a clinician to assess treatment efficacy, the risk of vision impairment in a patient's lifetime, and provide care according to individual need. Combinatorial analysis methods disclosed here intend to address the identified clinical needs. The subject invention covers algorithms for glaucoma detection consisting of conversion functions between test modalities, detection of local, regional, and global damage, agreement assessment, combined probability assessment, and a machine learning classifier; algorithms for glaucoma follow-up consisting of disease stage assessment, rate of change assessment, and progression event detection; and algorithms for combined analysis display.

In this document, a test modality refers to a diagnostic test, either structural or functional in nature, acquired with a diagnostic instrument such as HFA, Matrix, Stratus, Cirrus, GDx, and HRT. These instruments use perimetry, scanning laser polarimetry and optical coherence tomography as underlying technologies. Some instruments, such as Cirrus and HFA, are capable of providing several diagnostic tests or mutli-modality testing with the same instrument. Further, in some cases, multiple diagnostic tests are nested in a single data set, i.e., multiple diagnostic analyses can be performed on a single data set. An example of this is that one volumetric scan with Cirrus in the peripapillary region contains both the RNFL test and the ONH test. The RNFL test provides a quantitative measure of the nerve fiber layer thickness over the peripapillary region while the ONH test provides a quantitative measure of the nerve fiber thickness. Combining the analysis from RNFL and ONH tests is also covered under the scope of the subject invention, even if the only tests combined are the RNFL and ONH tests.

While the detailed descriptions below are mostly based on the combination of one structural test, specifically the RNFL measurement, and one functional test, specifically the visual field sensitivity measurement for glaucoma application, the methods can be adapted to other combinations of two or more diagnostic tests for glaucoma and/or to combinations of tests for other eye diseases. Applicable combinations include structure with structure, structure with function, or function with function. Any test modalities providing complementing and/or confirmatory assessment of disease damage may be combined. For example, RGG analysis is a quantitative measure of the thickness of the ganglion cell layer in the macula. Combinatorial analysis of the RNFL, ONH, and RGC assessment from OCT may be created to improve the overall clinical utility of the instrument for glaucoma management. Combination of the RNFL assessments acquired with OCT and GDx may help to differentiate RNFL tissue thickness change from axonal ultrastructural change. Further, the methods can be adapted to combinations of three or more test modalities, for example, a combination of the RNFL assessments by OCT and GDx and the sensitivity assessment by HFA. The diagram in FIG. 3 illustrates one exemplary approach to implement this idea where data from, perimetry, scanning laser polarimetry (SLP) and OCT are combined into a single RGC map that is used to generate both a diagnostic and stage index. The OCT include RNFL, ONH and macular inner layer analysis.

It should be understood that the embodiments, examples and descriptions in this document have been chosen and described in order to illustrate the principles of the invention and its practical applications and not as a definition of the invention. Modifications and variations of the invention will be apparent to those skilled in the art.

### Algorithms for Glaucoma Detection and Display

### Conversion functions

Conversion functions refer to mathematical models which convert spatial measurement of one or more test modalities to a selected spatial measurement so that the data from different test modalities can be presented in a common spatial distribution and measurement scale. The purpose of the conversion includes facilitating direct side-by-side comparison of test results from different modalities for easier interpretation and facilitating generation of combined test parameters through weighted averaging of two or more test modalities for further analysis. A conversion function may be from a structural test to a functional test or vice versa, and conversion functions may be established for local, regional, and global measurement parameters. Conversion functions may also combine two or more measurements from different diagnostic tests into a single diagnostic output.

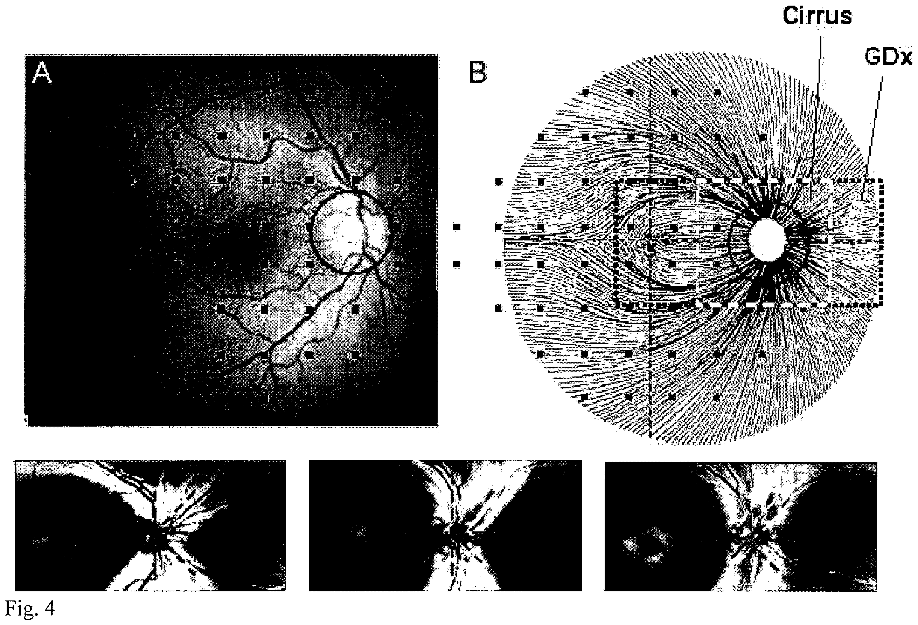

The conversion may be more straightforward between some test modalities with well-defined spatial correspondence, such as between peripapillary RNFL tests by OCT and GDx, central visual field sensitivity test by HFA and macular RGC assessment by OCT, and peripapillary RNFL test and ONH topography tested by OCT. Generating conversion functions between tests with more variable spatial correspondence may be more complex; for example, as shown in FIG. 4, between the central visual field test by HFA and the peripapillary RNFL test by GDx or Cirrus. Visual field test points indicated by dots on the top two images in the figure are distributed about the fovea and the RNFL measurements are distributed about the ONH as indicated by the white and gray dashed boxes for Cirrus and GDx respectively. The peripapillary RNFL distribution varies significantly across individual subjects. In this case, there is significant variation across subjects in both the spatial correspondence between the tests and the magnitude correspondence between the subjects. The bottom three scans of Figure 4 illustrate that there is significant variation across normal subjects in both the spatial correspondence between the tests and the magnitude correspondence between the subjects. The top two images illustrate that different diagnostic modalities have different spatial relationships to each other. Both of these facts complicate any combinatorial analysis. Furthermore, dynamic range differences between tests may add additional complexity to the conversion. Conversion functions for such test pairs may be established based on the average relationship across the population and factors contributing to the inter-subject variation should be identified and included in the conversion function to improve performance.

A publication described earlier (DF Garway-Heath et al. "Mapping the visual field to the optic disc in normal tension glaucoma eyes" Ophthalmology (2000) 127:674-680) established spatial correspondence between visual field regions and ONH sectors based on visually connecting locations of RNFL defect with locations of visual field scotoma, but no conversion function was developed for the regional measurements. A point-wise conversion model from a GDx VCC RNFL image to a 24-2 visual field sensitivity map was reported for the purpose of reducing variability in glaucoma follow-up through combined field (H Zhu et al. "Combining Structural and Functional Measurements to Improve Reproducibility of Follow Up Data in Glaucoma" Invest Ophthalmol Vis Sci (2009) Abs #2572). So far, little technical information has been published regarding this approach. The approach seems to be solely based on the GDx RNFL map as input without consideration for factors contributing to the inter-subject variation while relating the two tests.

Other publications referenced earlier attempt to correlate visual field sensitivity values on a log scale with RGC count on a linear scale (RS Harwerth et al. "Visual field defects and retinal ganglion cell losses in patients with glaucoma" Arch Ophthalmol (2006) 124:853-859, RS Harwerth et al. "Neural Losses Correlated with Visual Losses in Clinical Perimetry" Invest Ophthalmol Vis Sci (2004) 45:3152-3160 and WH Swanson et al. "Perimetric Defects and Ganglion Cell Damage: Interpreting Linear Relations Using a Two-Stage Neural Model" Invest Ophthalmol Vis Sci (2004) 45:466-472) or with RNFL thickness on a linear scale (DC Hood et al. "A Framework for Comparing Structural and Functional Measures of Glaucoma Damage" Progress in Retinal and Eye Research (2007) 26:688-710), where either no spatial conversion is required or existing regional spatial correspondence (DF Garway-Heath et al. "Mapping the visual field to the optic disc in normal tension glaucoma eyes" Ophthalmology (2000) 127:674-680) was employed.

In the subject invention, it is recognized that, to facilitate combinatorial analysis, conversion functions are desired to convert both the spatial distribution and measurement scale of a test modality to best match those of another test. Local or pixel-wise conversion, regional conversion, and global parameter conversion may all be needed to provide comprehensive combinatorial analyses for disease detection and/or follow-up. Furthermore, factors in addition to the measurement parameters of a test should be included in the conversion model to reduce conversion error.

The establishment of conversion functions requires a sufficiently large set of cross-sectional multi-modality clinical data (training data) with sufficiently complete coverage of the dynamic range of disease (i.e., from normal state through advanced disease stage without significant gap) and factors such as age and refraction, etc. The conversion functions should be optimized and evaluated based on a number of criteria, including, but not limited to: size of conversion error, dynamic range of the converted test, discriminating power of the converted test for disease detection, and test-retest variability of the converted test. To reduce the conversion error, additional parameters should be evaluated for inclusion in the conversion model, such as age, stage (e.g., MD and VFI in HFA or TSNIT average and NFI in GDx), image quality (e.g., intensity, contrast and TSS in GDx and signal-to-noise ratio in Cirrus), characteristics of the patient's eye (e.g., refraction, axial length, relative location of fovea to the optic disc center, retinal blood vessel pattern and orientation, and the shape and size of optic disc, etc.), and system parameters (e.g., GDx calibration parameters). Optimization of the conversion functions may be performed using a range of techniques, including machine learning, regression analysis, and principal components analysis.

One local structure-to-function conversion generates multidimensional outputs (HFA sensitivity values at 52 test locations of SITA 24-2) based on multidimensional inputs (GDx or Cirrus RNFL thickness values from the peripapillary region), using a machine learning method called Generalized Regression Networks (GRNN). The GRNN contains a radial basis layer and a special linear layer and is often used in the neural network training to create a regression model used for multidimensional input to multidimensional output mapping. The implementation of this method is available in Matlab Neural Network Toolbox. During training, the adjustable parameters of the network (weights) are set so as to minimize the average error between the actual network output and the desired output over the target training set.

The GRNN is implemented in Matlab through the function "newgrnn (P,T,S)", where P is matrix consisting of input vectors (GDx or Cirrus measurements), T is a matrix consisting of target vectors (HFA measurements), and S is the spread of radial basis functions. This function returns a generalized regression model. The function prototype is defined as follows, model = newgrnn (P, T, S); The larger the S, the smoother the function approximation will be. A small S value can be used to fit data very closely, and a larger S can be used to fit the data more smoothly. To fit data closely, we used S smaller than the typical distance between input vectors. Once a model is created, output map may be generated using T1 = sim (model, P1); PI is a set of test or validation input data (GDx or Cirrus) and T1 is corresponding output maps (converted field).

The preprocessing steps associated with the input vectors (P) based on GDx measurement start with the full RNFL map and include: (1); preferred but not required, a smoothing algorithm is applied to remove the blood vessels (2); the image is laterally translated to center on the ONH and the angle of rotation of the line connecting the center of fovea and center of ONH is determined (3); the image is rotated about the ONH center so that the line connecting the fovea and ONH centers is horizontal (4); an annular region with inner radius of 23 pixels and outer radius of 48 pixels, centered on the ONH, is extracted as the region of interest for input vector (5); optionally, the region may be divided to superior and inferior hemi-fields to train two separate models (6); preferred but not required, the input vectors are scaled to the range of [-1 1] (7); optionally, the input vector can be converted from linear scale to log scale (8).

The preprocessing steps associated with preparing the target vectors (T) are simple and must be consistent with the input vector configuration. It starts with the sensitivity values of the 52 test locations and followed by 3 options of pre-processing: the 52-points may be divided to superior and inferior hemi-fields to train two separate models, if the same step is applied to the input vectors; the target vectors may be scaled to the range of [-1 1], if the same step is applied to the input vectors; the target vectors is converted from log scale to linear scale, if the input vectors are in linear scale.

Multiple conversion models (with different preprocessing configurations) based on GDxECC and HFA combination were developed and tested, and the preferred model identified based on converted ECC normative database distribution and results of the testing data set. Four models selected for their attributes and performance:
Model 1_0_1_1_3 (smoothing over blood vessel, full field, linear scale, scaling, and spread of 3)
Model 1_0_1_1_2.5 (smoothing over blood vessel, full field, linear scale, scaling, and spread of 2.5)
Model 1_0_2_0_50 (smoothing over blood vessel, full field, log scale, no scaling, and spread of 50)
Model 1_1_1_1_2 (smoothing over blood vessel, hemi field, linear scale, scaling, and spread of 2)

### Implementation of STATPAC-like normative data analysis for the converted field

HFA Ensemble software was modified to perform STATPAC-like analysis (comparison to normal limits) on the converted field. The analysis must be performed in a way that is conversion model specific because the normative limits are different for different models. The normative limits for mean deviation (MD), PSD, Total deviation, and Pattern deviation were implemented for each of the 4 ECC conversion models. In addition to these parameters, Visual Field Index (VFI) is also calculated for the converted field for inclusion in feasibility investigation of stage index calculations. The most relevant outputs of the Ensemble are MD and p-value, PSD and p-value, VFI, Total Deviation Probability Plot, and Pattern Deviation Probability Plot. The converted fields of the testing data set for each of the 4 ECC models were processed and exported for further analysis to assess model performance.

Mean Deviation (MD) - is a weighted average deviation from the normal reference field. MD estimates the uniform part of the deviation, and may be interpreted as a measure of deviation of height (of a person's field of vision from what is the statistical normal). Total Deviation takes the raw data results for each test point of an HFA exam and compares the results against an established age-corrected normal. The deviation is the difference between what is "statistically" normal for a particular test point and the measured value at this test point. If the patient saw better than normal, the result will be a positive deviation, if the patient saw worse, then the deviation will be negative. From these deviations a probability is determined which indicates whether the deviations are non-significant or if significant, how much (is this deviation present in < 5% of the population?, < 2% of the population?, etc). Pattern Deviation is, in simple terms, an offset - up or down - in the Total Deviation. The amount of offset is called the elevator. This shifting of the Total Deviation field filters out noise caused by such things as cataracts, small pupils, or "supernormal" vision making the results more sensitive to localized scotomas. As with Total Deviation, from these pattern deviations a probability can be determined indicating how significant this deviation is.

The visual field index (VFI) is a weighted summary of the effect of glaucomatous loss on the visual field represented as a percentage. Bengtsson and Heijl described in 2008 the basis for the Visual Field Index. Initially called the Glaucoma Progression Index (GPI), this index utilizes data from the pattern deviation probability maps and is incorporated into the new VFI graphical analysis in the GPA 2 software. To avoid effects of cataract, the pattern deviation probability maps are used to identify test points having normal sensitivity and those demonstrating relative loss. Test points having threshold sensitivities within normal limits on the pattern deviation probability maps are considered normal and are scored at 100% sensitivity. Test points having absolute defects, defined as measured threshold sensitivities of less than 0 dB, are scored at 0% sensitivity. Points with significantly depressed sensitivity, but not perimetrically blind (relative loss), are identified as test points with sensitivities depressed below the p < 0.05 significance limits in the pattern deviation map. The sensitivity at these points are scored in percent. The scores are weighted according to how far a given test point is from the fovea. The weights decrease with increasing eccentricity. The VFI is the mean of all weighted scores in percent. The effects of this weighting procedure on the VFI are most pronounced in the parafoveal region and less pronounced peripherally. Linear regression analysis can be used to determine the rate of change in VFI.

### Detection of local, regional, and global damage

It is recognized that there is wide range of morphological variation in structural and functional damage caused by glaucoma; damage may occur diffusely, localized, or mixed and locations of damage vary from eye to eye. For damage to be detected without longitudinal follow-up, the level of damage must exceed the limits of the distribution for normal eyes. The normative limits include test-retest variability and subject variability of a normal population and are usually wider for local parameters than global parameters. Therefore, in accordance with the subject invention, it is desirable for the combinatorial analyses to analyze multi-modality test data with varying spatial resolution in order to capture global, regional, and/or local damages to the structure and function of the eye essential to early detection of the disease. The multi-modal combinatorial analyses are novel and essential to one aspect of our invention.

Global damage is best measured with global parameters such as temporal-superior-nasal-inferior-temporal (TSNIT) Average in GDx and OCT, or mean deviation (MD) and pattern standard deviation (PSD) in HFA. Global parameters have less (or the least) test-retest variability and inter-subject variability and are more sensitive to small levels of damage covering a large area.

Regional damage is best measured with regional parameters covering areas similar to the damage. Regional parameters have higher test-retest variability and inter-subject variability than global parameters, and the level of damage detectable is likely higher than that of diffuse damage. The 6 regions defined in Garway-Heath map (DF Garway-Heath et al. "Mapping the visual field to the optic disc in normal tension glaucoma eyes" Ophthalmology (2000) 127:674-680) and the 10 regions defined in the GHT test are examples of regional parameters in HFA; the 6 sectors of the ONH and TSNIT plot (DF Garway-Heath et al. "Mapping the visual field to the optic disc in normal tension glaucoma eyes" Ophthalmology (2000) 127:674-680), clock hour measurements, and quadrant measurements are examples of regional parameters in GDx and/or OCT.

Small local damage is best measured with local parameters consisting of individual pixels or super pixels of structural measurements and individual test points in functional measurements. The RNFL image in GDx and OCT or visual field sensitivity map in HFA are examples of local parameters. Local parameters have higher test-retest variability and inter-subject variability, and the level of small local damage detectable is likely higher than those of diffuse damage and regional damage.

### Combinatorial Analysis Approaches

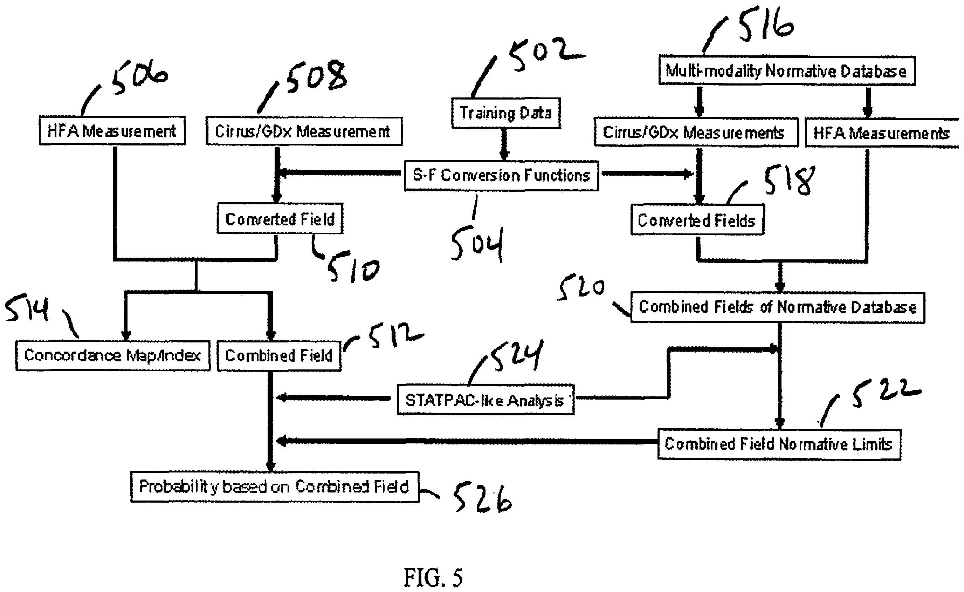

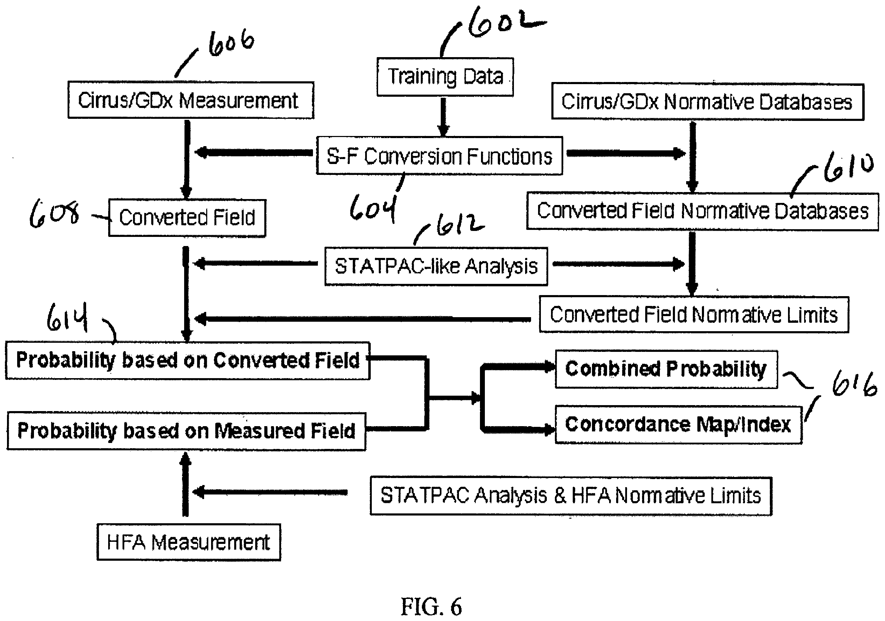

In the subject invention, two alternative approaches are identified for the implementation of the multi-modal combinatorial analyses. One approach, illustrated in FIG. 5, is to combine the multi-modality tests into one test and compare the combined test with multi-modality normative limits to assess the probability of the combined test being within its normal range. Alternatively, illustrated in FIG. 6, each test modality can be analyzed separately and the probabilities being within the normal range of each individual test are then combined to assess the multi-modality combined probability. The two approaches are illustrated in FIGS. 5 & 6 based on converting the spatial distribution and scale of RNFL test data from OCT and/or GDx measurements to visual field sensitivity data but the opposite conversion could be taken as well. Black color indicates initial input, blue color indicates intermediate results, red color indicates outputs, dotted lines and arrows indicate alternative or optional path.

The steps of FIG. 5 include:
1) Collecting training data with visual field testing (HFA) and OCT (Cirrus) and/or GDx from subjects across the dynamic range of glaucoma testing (normal through advanced glaucoma); (step 502)
2) Developing structure to function (S-to-F) conversion functions (point-wise, regional, and global) for the Cirrus and/or GDx data using the training data set; (step 504)
3) Acquiring and analyzing image data from HFA and Cirrus and/or GDx from a particular subject; (steps 506/508)
4) Applying the S-to-F conversion function to the analyzed Cirrus and/or GDx subject data; (step 510)
5) Generating combined visual field sensitivity measurements based on the weighted mean of the measured visual field sensitivity from the HFA measurement and the RNFL-converted visual field sensitivity from OCT and//or GDx measurements and provide agreement assessment through a concordance map or index; (steps 512/514)
6) Collecting multi-modality data in a normative database; (step 516)
7) For the Cirrus/GDx measurements in the database, applying the S-to-F conversion to generate converted fields; (step 518)
8) Generating combined fields of the normative database from the HFA data and the converted RNFL data from Cirrus and/or GDx; (step 520)
9) Establishing normative limits to facilitate STATPAC-like analysis for combined visual field sensitivity measurement; and (step 522)
10) Running STATPAC-like analysis on the combined field to provide combined probability assessment for local, regional, and global parameters (steps 524/526).
Similarly, the steps in FIG. 6 include:
1) Collecting training data with visual field testing (HFA) and OCT (Cirrus) and/or GDx from subjects across the dynamic range of glaucoma testing (normal through advanced glaucoma); (step 602)
2) Developing S-to-F conversion functions (point-wise, regional, and global) for the Cirrus and/or GDx data using the training data set; (step 604)
3) Acquiring and analyzing HFA Cirrus and/or GDx data from an individual subject; (step 606)
4) Applying the S-to-F conversion function to the analyzed Cirrus and/or GDx subject data to generate a visual field; (step 608)
5) Converting existing structural normative database to visual field space with the S-to-F conversion functions to facilitate STATPAC-like analysis; (step 610)
6) Running STATPAC-like analysis on the converted field using the converted field normative database; (step 612)
7) Establishing normative limits on the Cirrus and/or GDx data and determining an individual probability for the structural data; (step 614) and
8) Comparing the results with STATPAC analysis performed on measured visual field to provide agreement assessment through a concordance map or index and optionally, combined probability assessment (step 616).

Both approaches require that the analysis from the multi-modality tests be first converted to a common spatial distribution and measurement scale using conversion functions. The approach in FIG. 5 requires a normative database consisting of multi-modality test data to be available while the approach in FIG. 6 could make use of existing normative databases of individual modalities, converted to establish normative limits for the converted tests. For both approaches, there are two alternatives to derive regional and global parameters for the converted test, directly convert from regional and global parameters of the original tests using regional and global conversion functions or derive the parameter from the converted test with higher spatial resolution (e.g., regional parameters of converted visual field derived directly from point-wise converted field). Due to the likely higher inter-subject variation in localized (point-wise) conversion, the direct regional and global conversion may be preferred.

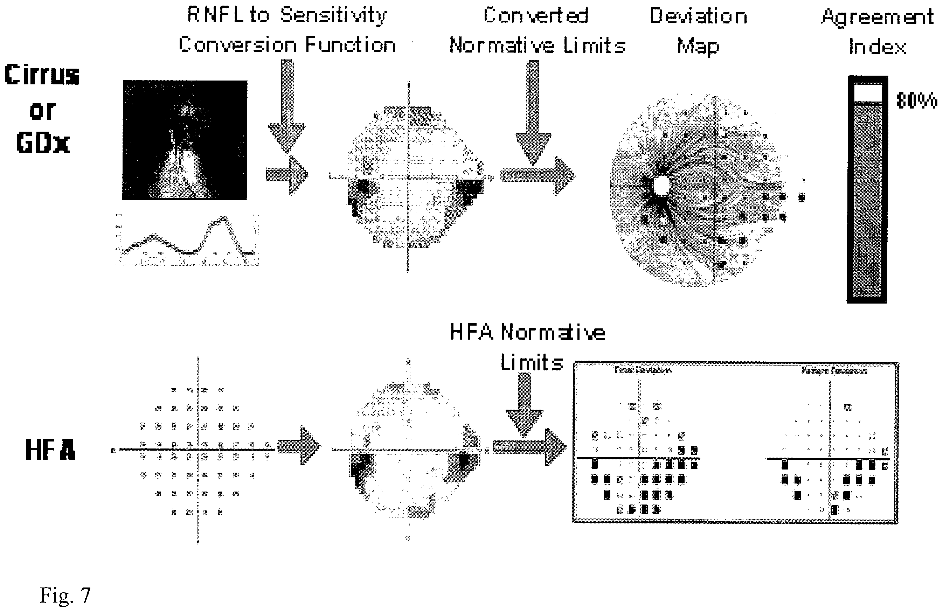

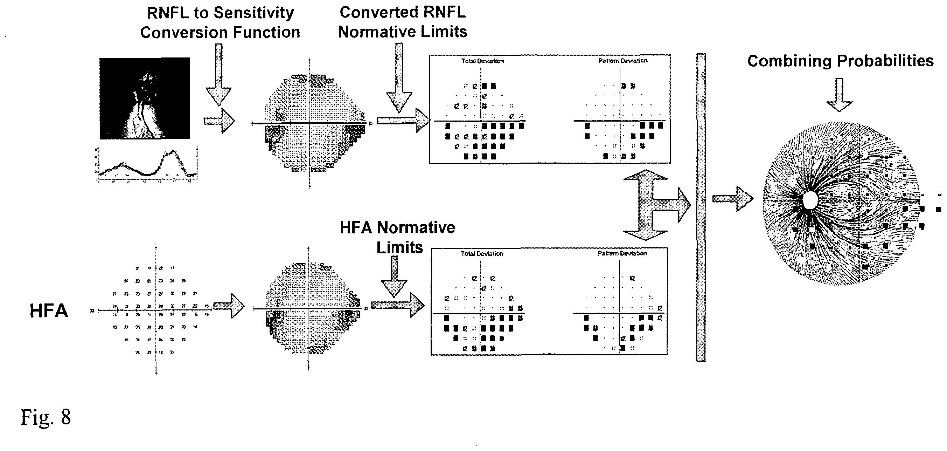

To detect both diffuse and local glaucoma damage, multi-modality data should be analyzed based on a combination of global analysis, regional analysis, and localized analysis. For example, as illustrated in FIGS. 7 and 8, if the approach in FIG. 6 is selected, the localized analysis involves converting a RNFL measurement from OCT or GDx to a pseudo HFA SITA 24-2 format sensitivity map with a point-wise conversion model, establishing normative limits for the converted field from the existing RNFL normative database, applying STATPAC-like analysis for the converted field to generate deviation plots and probability plots for the converted field, providing side-by-side comparison of test results based on converted field and measured field, assessing agreement (FIG. 7), and/or assessing combined probability if desired (FIG. 8). Displaying structural test results from OCT and/or GDx in a format similar to that of measured visual field data facilitates more straightforward interpretation of multi-modality test results. The agreement index and combined probability help to further simplify clinical interpretation of multi-modality data and improve consistency of interpretation across observers.

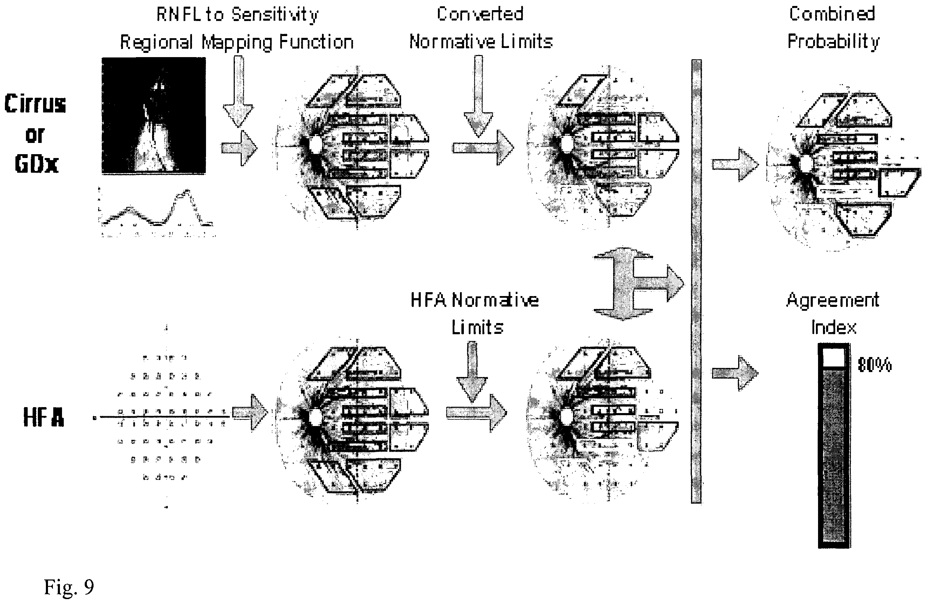

The regional analysis involves conversion of an RNFL measurement to regional visual field sensitivity. The definition of regions may be based on GHT zones as illustrated in FIG. 9 or Garway-Heath zones as shown in FIG. 2. For the approach in FIG. 6, normative limits for the regional measurements need to be established for the measured visual field and the predicted visual field respectively. The steps are similar as those for local analysis as illustrated in FIG. 9 where GHT zones were selected as the basis for the regional measurements. The regional analysis could be based on Garway-Heath zones or other definitions of measurement region clinically or anatomically sensible. The regional analysis results may be displayed based on functional definition of regions (FIG. 9) or corresponding structural regions.

Global parameters may be derived from the converted pseudo visual field sensitivity map or from direct conversion from RNFL global parameters. Whichever method yields lower conversion error should be employed. Analysis of global parameters requires corresponding normative limits to be established.

It is to be understood that the multi-modal combinatorial analysis doesn't have to have more than two analysis modes. It may be sufficient to have, for examples, an integration of global analysis with regional analysis or an integration of global analysis with local analysis.

The structure-to-function conversion functions (pointwise, regional, and global) should be established with a sufficiently large set of cross-sectional training data independent of the normative database for the establishment of combinatorial analysis normative limits. The collection of multi-modality data for generating a normative database should avoid potential bias in subject enrollment towards any one of the tests included in the combinatorial analysis.

### Machine learning classifier for glaucoma detection

Multi-modality machine learning classification (MLC) facilitates the much desired simplification of clinical interpretation for disease detection. Multi-modality clinical data is required for the training of the machine learning classifier. The data set should consist of both normal subjects and glaucoma subjects with enrollment criteria unbiased by the modalities being combined.

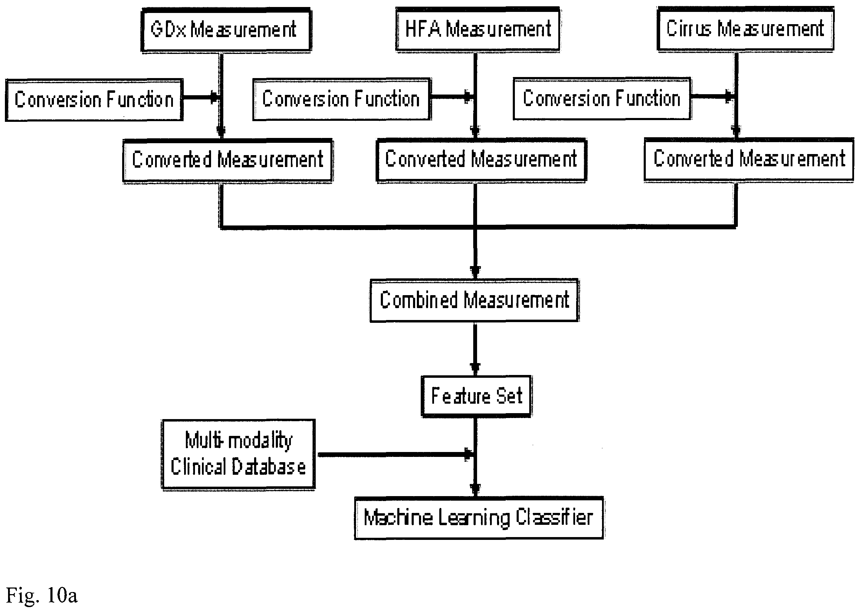

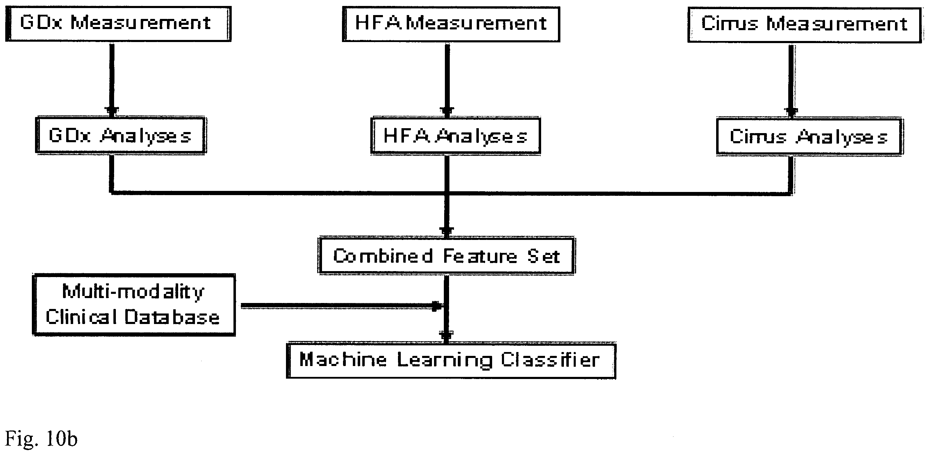

The steps for the development of machine learning classifier are shown in FIGS. 10a and 10b and include collecting multi-modality clinical data based on enrollment criteria unbiased by the modalities being combined; the subjects should include normals and patients. One approach is to normalize and map structural measurements and functional measurements to a common scale and distribution, then combine the measurements, and derive input feature set for MLC training from the combined measurement. Alternatively, it is possible to construct an input feature set for MLC training from the features of individual modalities.

As illustrated in FIG. 10a, the input parameters (feature set) for the machine learning classifier may consist of global, regional, and local parameters, or their corresponding probability values derived from the combined measurement using conversion functions. This approach may require establishment of normative limits for the combined test, and may not utilize all of the existing analyses in individual modalities.

Alternatively as shown in FIG. 10b, the input parameters (feature set) for the machine learning classifier may consist of global, regional, and local parameters directly obtained from individual modalities in their own measurement units (e.g. sensitivity values or RNFL thickness values), in deviations from age-corrected normal values, or in probability values based on comparison with their respective normative limits.

The output of the machine learning classifier could be a classification with three categories (e.g. Within Normal Limits, Borderline, and Outside Normal Limits) or a continuous index (e.g., value ranging from 0 to 100). A threshold may be set for the index according to the desired balance of specificity and sensitivity. Presumably the thresholded index has improved sensitivity at a given specificity, or improved specificity at a given sensitivity. Therefore for an individual, it can be considered as confirming (or refuting) the individual test, if a previously undetected case is now detected, or a previous false positive is now correctly identified as not having the pathology.

In one embodiment of the MLC, Support Vector Machines (SVM) are used to learn the mapping of Cirrus measurements of ONH and RNFL to glaucomatous damage as determined by a clinical site using visual field measurements. SVMs take input *n*-d feature vectors and create linear partitions of that space that maximize the margin separating the two classes from that hyperplane. It is a powerful technique that not only improves the ability to generalize to unseen data by maximizing the margin (the buffer between an object of one class, the hyperplane and an object of another class), but casts the input data into a higher-dimensional space to do so, where there is no limit on the dimensionality of the resultant feature space that the SVM chooses to use. So although the SVM is linear in its creation of a hyperplane, it is non-linear in the mapping to a higher dimension where it then finds that hyperplane.

Ahead of building the SVM classifier, it is beneficial to look at each feature in isolation to estimate its ability to discriminate. One measure could be the variance of the feature across the population, factoring in its classification. The F-score does this by summing the variances for the class means with respect to the overall mean. It measures the discriminatory ability of two sets of numbers (one from each class), giving the likelihood of a feature's ability to discriminate among those classes.

The input training has its features scaled to a given range (-1/+1), for normalization purposes (the input ranges are stored for application to unseen data). We can then perform a grid search across the two SVM parameters C and Gamma, which control the nature of the function applied to cast the input parameters into a higher dimensional space. A brief grid search uses 10-fold cross validation to determine a sensible parameter range. The SVM returns a distance from the hyperplane that separated the two classes during the training stage. It is a maximum margin classifier, so it creates a buffer, the margin, to ensure that it is not just fitting a plane, rather partitioning the data in a more meaningful way. The natural result then of classifying an observation is to return a distance from the hyperplane itself. A distance of zero is right on the border. As the distance is negative, that means we have a negative classification (by convention), which for us is a normal classification; positive values implies positive classification, which is Glaucoma. A nominal decision threshold is therefore zero.

The performance using four different sets of features was evaluated. We concluded that this feature is capable of producing extremely high AUCs, and performance in the case where the demographic was certainly captured is excellent.

### Reliability of individual test

Reliability of an individual test should be assessed and taken into account in combinatorial analysis. A less reliable test should have lower weight in calculating the combined measurement or the combined probability. An unreliable test should be recognized and excluded from the combinatorial analysis.

The confounding factors affecting test reliability vary with modalities. In HFA, measurement artifacts may be caused by droopy lids, cataracts, correction lens artifacts, and learning effects; in GDx, measurement artifacts may be caused by atypical scans, peripapillary atrophy (PPA), poor fixation, and poor corneal compensation; in Cirrus, measurement artifacts may be caused by low signal-to-noise ratio, eye motion, and segmentation failure.

Algorithms can be developed based on machine learning with appropriate clinical data to automatically recognize artifacts and assess test reliability. Test reliability could then be included in the combined analysis. Test reliability may be assessed locally, regionally, or globally, based on need.

As an example, if a Cirrus scan indicates loss of RNFL, while an HFA result indicates that the patient's visual function is within normal limits, the algorithm may note that the signal strength is lower than optimal, which could contribute to a low RNFL measurement. In this case, the algorithm would refute the Cirrus result (thin RNFL). As an alternate example, if a Cirrus scan indicates RNFL within normal limits, while an HFA result indicates that the patient's visual function is outside normal limits, or depressed in some area, the algorithm may note that the test reliability is lower than optimal, or may note that the test reliability criterion was low, in which case the algorithm refutes the HFA finding.

When reliability of an individual test is unknown, the combined analysis may be based on equal weighting of the tests being combined. Furthermore, the dynamic range of an individual test may be established based on clinical data and tests may be combined with appropriate weights assigned based on the known dynamic range. If a subject falls outside the dynamic range of a given test, less weight should be assigned to the test relative to other tests by which the subject is within the dynamic range. As an example, the Cirrus RNFL measurement does not change much as disease progresses from severe to very severe glaucoma. RNFL measurements at or below 50 µm may be weighted such that HFA results dominate staging in this range. When dynamic range of the tests to be combined is unknown, the combinatorial analysis may be based on equal weight for tests being combined. Alternatively, if a large set of clinical data is available, machine learning may be an approach to optimize the weights for the combined analysis.

### Algorithms for Glaucoma Follow-Up and Display

It is recognized in the subject invention that disease stage assessment is essential in initial examination and follow-up of glaucoma. At a minimum, a global function or stage index should be provided, and if desired, regional or even local stage indices should also be provided. Combining multi-modality tests could potentially improve stage assessment. Stage indices obtained in longitudinal follow-up seem appropriate parameters for assessing rate of change and detection of progression.

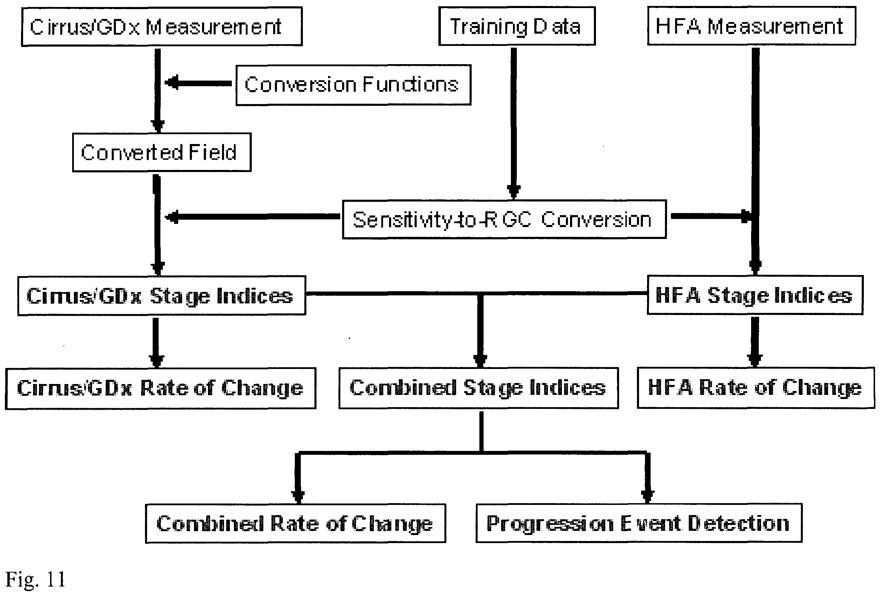

One exemplary embodiment is illustrated in FIG. 11. Similar to the glaucoma detection examples previously discussed, the multi-modality tests are converted to a common spatial distribution and scale using conversion functions. For stage assessment, the common scale is preferred to be proportional to RGC count. The conversion from a measurement scale to RGC count may be based on published clinical studies (RS Harwerth et al. "Visual field defects and retinal ganglion cell losses in patients with glaucoma" Arch Ophthalmol (2006) 124:853-859, RS Harwerth et al. "Neural Losses Correlated with Visual Losses in Clinical Perimetry" Invest Ophthalmol Vis Sci (2004) 45:3152-3160, DC Hood et al. "A Framework for Comparing Structural and Functional Measures of Glaucoma Damage" Progress in Retinal and Eye Research (2007) 26:688-710 and WH Swanson et al. "Perimetric Defects and Ganglion Cell Damage: Interpreting Linear Relations Using a Two-Stage Neural Model " Invest Ophthalmol Vis Sci (2004) 45:466-472) or based on appropriate training data. Ideally, the conversion would yield a spatial distribution of RGC count. It may be of interest to calculate a stage index for each modality respectively and compare indices for agreement. Combined stage indices may be desired and can be calculated from combining the individual modality's indices. The implementation should facilitate options of assessing global, regional, and local indices.

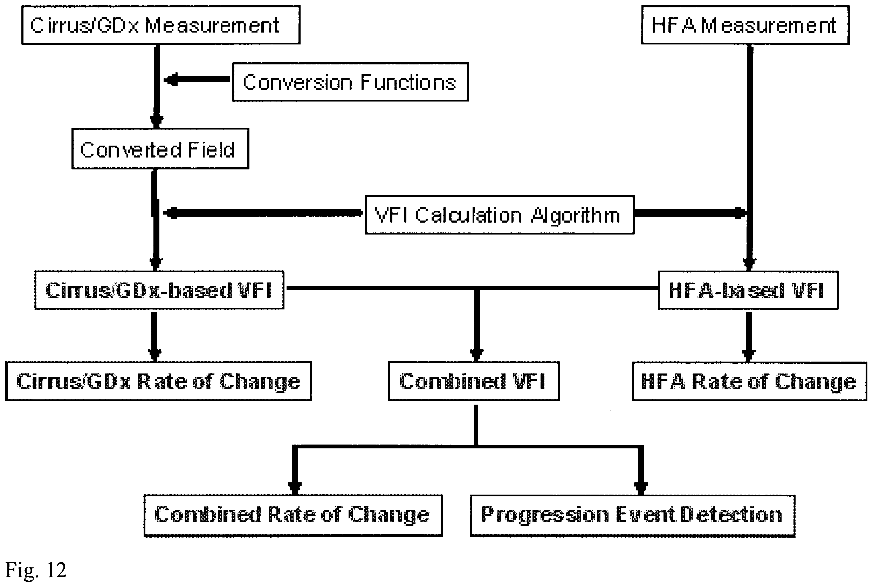

An alternative to conversion to RGC count is to utilize the visual field index (VFI) calculation in HFA which has been introduced in clinical practice as a stage index, as shown in FIG. 12. VFI is currently implemented as a global index

### Key Elements for Combinatorial Analysis Report

After performing the analysis, it is desirable to have an integrated report to simplify interpretation and to improve workflow. The report should include glaucoma test data and treatment data, provide a summary of glaucoma detection (FIGS. 7-8), and provide trend plots of stage index and treatment data to facilitate efficient assessment of individual risk for vision impairment and treatment efficacy.

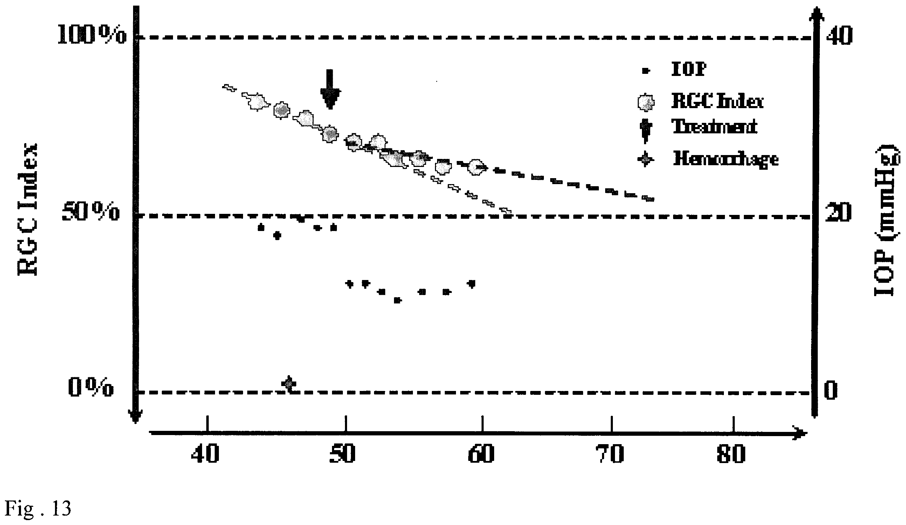

An exemplary embodiment of trend plot is illustrated in FIG. 13 in which glaucoma test data (Stage index over time and trend) and glaucoma treatment data (IOP over time) are displayed in parallel on the same graphical image as a function of time. A doctor can easily assess whether the IOP-lowering target has been achieved following the treatment and whether the IOP lowering has the desired effect in slowing down disease progression from this display. To be noted, the scale for the disease stage index may be displayed in log scale, if it is deemed clinically meaningful.

It should be understood that the embodiments, examples and descriptions have been chosen and described in order to illustrate the principles of the invention and its practical applications and not as a definition of the invention. Modifications and variations of the invention will be apparent to those skilled in the art. The scope of the invention is defined by the claims, which includes known equivalents and unforeseeable equivalents at the time of filing of this application.

### DEFINITIONS, ACRONYMS, AND ABBREVIATIONS

- ECC:: Enhanced corneal compensation imaging mode in GDx
- EMR:: Electronic medical records
- ERG:: Electroretinography
- FDT:: Frequency-doubling technology for visual function testing
- GDx:: Scanning laser polarimetry system manufactured by Carl Zeiss Meditec Inc. for testing the retinal nerve fiber layer
- GHT:: Glaucoma hemifield test in HFA
- GPA:: Guided progression analysis software, available in both GDx and HFA
- GPS:: Glaucoma probability score, a HRT machine learning classifier
- HFA:: Humphrey field analyzer made by Carl Zeiss Meditec Inc. for testing visual field sensitivity
- HEP:: Heidelberg Edge Perimeter for testing visual function
- HRT:: Heidelberg retinal tomography system for optic nerve head topography
- LDF:: Linear discriminate function
- Matrix:: Field analyzer based on FDT made by Carl Zeiss Meditec Inc.
- MD:: Mean deviation, a visual field index in HFA
- NFI:: Nerve fiber indicator, a GDx machine learning classifier
- OCT:: Optical coherence tomography system for retina made by Carl Zeiss Meditec Inc.
- ONH:: Optic nerve head of the human eye
- PSD:: Pattern standard deviation, a visual field index in HFA
- PPA:: Peripapillary atrophy
- RGC:: Retinal ganglion cell
- RNFL:: Retinal nerve fiber layer
- SAP:: Standard automated perimetry
- SITA:: Swedish interactive testing algorithms for visual field testing in HFA, including SITA Fast and SITA Standard
- SAP:: Standard automated perimetry
- SLP:: Scanning laser polarimetry system for testing the retinal nerve fiber layer
- STATPAC:: Analysis software implemented in HFA to identify visual fields that fall outside normal range or to identify visual field progression
- SWAP:: Short wavelength automated perimetry
- TCA:: Topographic change analysis in HRT
- VCC:: Variable corneal compensation imaging mode in GDx

### REFERENCES

RS Harwerth et al. "Visual field defects and retinal ganglion cell losses in patients with glaucoma" Arch Ophthalmol (2006) 124:853-859.
HA Quigley et al. "Retinal ganglion cell atrophy correlated with automated perimetry in human eyes with glaucoma" Am J Ophthalmol (1989) 107:453-464.
RS Harwerth et al. "The relationship between nerve fiber layer and perimetry measurements" Invest Ophthalmol Vis Sci (2007) 48:763-773.
DF Garway-Heath et al. "Mapping the visual field to the optic disc in normal tension glaucoma eyes" Ophthalmology (2000) 127:674-680.
TA Beltagi et al. "Retinal nerve fiber layer thickness measured with optical coherence tomography is related to visual function in glaucomatous eyes" Ophthalmology (2003) 110:2185-2191
NJ Reus et al. "The relationship between standard automated perimetry and GDx VCC measurements" Invest Ophthalmol Vis Sci (2004) 45:840-845.
LA Kerrigan-Baumrind et al. "Number of Ganglion Cells in Glaucoma Eyes Compared with Threshold Visual Field Tests in the Same Persons" Invest Ophthalmol Vis Sci (2000) 41:741-748.
LK Singh et al. "Optic Nerve Head and Retinal Nerve Fiber Layer Analysis - A Report by the American Academy of Ophthalmology" Ophthalmology (2007) 114:1937-1949.
MF Delgado et al. "Automated perimetry: a report by the American Academy of Ophthalmology" Ophthalmology (2002) 109:2362-2374.
FA Medeiros et al. "Comparison of the GDx VCC Scanning Laser Polarimeter, HRT II Confocal Scanning Laser Ophthalmoscope, and Stratus OCT Optical Coherence Tomograph for the Detection of Glaucoma" Arch Ophthalmol (2004) 122:827-837.
NJ Reus et al. "Scanning Laser Polarimetry of the Retinal Nerve Fiber Layer in Perimetrically Unaffected Eyes of Glaucoma Patients.Ophthalmology" (2004) 111:2199-2203.
BC Chauhan et al. "Optic Disc and Visual Field Changes in a Prospective Longitudinal Study of Patients With Glaucoma - Comparison of Scanning Laser Tomography With Conventional Perimetry and Optic Disc Photography" Arch Ophthalmol (2001)119:1492-1499.
P Artes et al. "Longitudinal changes in the visual field and optic disc in glaucoma" Progress in Retinal and Eye Research (2005) 24:333-354
X-R Huang et al. "Microtubules Contribute to the Birefringence of the Retinal Nerve Fiber Layer" Invest Ophthalmol Vis Sci (2005) 46:4588-4593.
B Fortune et al. "Retinal Nerve Fiber Layer Birefringence Declines Prior to Thickness After Onset of Experimental Glaucoma or Optic Nerve Transection in Non-Human Primates" Invest Ophthalmol Vis Sci (Suppl) (2008) 49: abstract #3761.
E. Gotzinger et al. Retinal Nerve Fiber Layer Birefringence of Healthy and Glaucomatous Eyes Measured with Polarization Sensitive Spectral Domain OCT" Invest Ophthalmol Vis Sci (Suppl) (2008) 49: abstract #3762.
FK Horn et al. "Correlation Between Local Glaucomatous Visual Field Defects and Loss of the Nerve Fiber Layer Thickness Measured with Polarimetry (GDx) and Spectral Domain OCT" Invest Ophthalmol Vis Sci (Suppl) (2008) 49: abstract #732.
DR Anderson Automated Static Perimetry St. Louis: Mosby-Year Book 1992.
RS Harwerth et al. "Neural Losses Correlated with Visual Losses in Clinical Perimetry" Invest Ophthalmol Vis Sci (2004) 45:3152-3160.
NG Strouthidis et al. "Structure and Function in Glaucoma: The relationship between a Functional Visual Field Map and an Anatomic Retinal Map" Invest Ophthalmol Vis Sci (2006) 47:5356-5362.
DC Hood et al. "A Framework for Comparing Structural and Functional Measures of Glaucoma Damage" Progress in Retinal and Eye Research (2007) 26:688-710.
N Shah et al. "Combining Structural and Functional Testing For Glaucoma detection" Ophthalmology (2006) 113:1593-1602.
CY Mardin et al. "Improving Glaucoma Diagnosis by the Combination of Perimetry and HRT Measurements" J Glaucoma (2006) 15:299-305.
JL Hougaard et al. "Modeling the Normal Retinal Nerve Fiber Layer Thickness as Measured by Stratus Optical Coherence Tomography" Graefe's Arch Clin Exp Ophthalmol (2006) 244:1607-1614.
HA Quigley et al. "Optic Nerve Damage in Human Glaucoma. III. Quantitative correlation of Nerve Fiber Loss and Visual Field Defect in Glaucoma, ischemic Optic Neuropathy, Papilledema, and Toxic Neuropathy" Arch Ophthalmol (1982) 100:135-146.
HA Quigley et al. "Retinal Ganglion Cell Atrophy Correlated with Automated Perimetry in Human Eyes with Glaucoma" AM J Ophthalmol (1989) 107:453-464.
WH Swanson et al. "Perimetric Defects and Ganglion Cell Damage: Interpreting Linear Relations Using a Two-Stage Neural Model" Invest Ophthalmol Vis Sci (2004) 45:466-472.
H Zhu et al. "Combining Structural and Functional Measurements to Improve Reproducibility of Follow Up Data in Glaucoma" Invest Ophthalmol Vis Sci (2009) Abs #2572.
Bengtsson, B et al. "A visual field index for calculation of glaucoma rate of progression" Am J Ophthalmol (2008) 145:343-353.
Zhu, H et al. "Predicting Visual Function from the Measurements of Retinal
Nerve Fibre Layer Structure" Invest Ophthalmol Vis Sci. (2010) May 26. [Epub ahead
of print] PubMed PMID: 20505207.

## Claims

1. A method of analyzing the degree of abnormality of retinal tissue in a patient's eye comprising:
collecting measurements of the retina of the patient's eye using one diagnostic test from one diagnostic instrument;
applying a conversion function to the measurements that modifies the spatial distribution and measurement scale of the collected measurements to match the spatial distribution and measurement scale of a second diagnostic test collected with a second diagnostic instrument;
comparing the functional output for the patient to a probability distribution created from measurements on normal subjects to indicate a likelihood of normality; and
displaying the state of the functional output relative to normal on a graphical display.

2. A method as recited in claim 1, further comprising displaying the output of the function.

3. A method as recited in claim 1, wherein the conversion function is configured to maximize the similarity of the results of the two diagnostic tests across the patient population.

4. A method as recited in claim 1, wherein the diagnostic tests include one structural test and one functional test of the eye.

5. A method as recited in claim 1, wherein at least one of the diagnostic tests is selected from the group consisting of: visual field testing, RNFL analysis, ONH analysis, ganglion cell analysis, and macular inner retinal thickness.

6. A method of analyzing the degree of abnormality of a retina in a patient's eye comprising:
collecting two or more measurements of the patient's eye using two different diagnostic tests collected using one or more diagnostic instruments;
applying a conversion function to the measurements from one of the diagnostic tests that modifies the spatial distribution and measurement scale of the measurements to match the spatial distribution and measurement scale of the second diagnostic test;
combining the converted measurements from the first diagnostic test and the measurements from the second diagnostic test to generate an output that is optimized to discriminate between normal and disease;
comparing the combined output for the patient to a probability distribution created from measurements on normal subjects to indicate a likelihood of normality; and
displaying the state of the functional output relative to normal on a graphical display.

7. A method as recited in claim 6, further comprising displaying the output of the function.

8. A method as recited in claim 6, wherein the diagnostic tests include one structural test and one functional test of the eye.

9. A method as recited in claim 6, wherein at least one of the diagnostic tests is selected from the group consisting of: visual field testing, RNFL analysis, ONH analysis, ganglion cell analysis, and macular inner retinal thickness.

10. A method as recited in claim 6, wherein the one or more diagnostic instruments are selected from: perimetry, scanning laser polarimetry, and optical coherence tomography (OCT).

11. A method as recited in claim 10, wherein the two or more measurements are made using the same diagnostic instrument.

12. A method as recited in claim 6, wherein the output of the function is in the same form as one of the inputs.

13. A method as recited in claim 6, wherein the inputs to the function are weighted according to the reliability of the individual diagnostic tests.

## Patentansprüche

1. Verfahren zur Analyse des Anomaliegrades von Netzhautgewebe im Auge eines Patienten, umfassend:
Erheben von Messungen der Netzhaut des Auges des Patienten mithilfe einer diagnostischen Untersuchung durch ein Diagnoseinstrument;
Anwenden einer Umwandlungsfunktion auf die Messungen, die die räumliche Verteilung und den Maßstab der erhobenen Messungen verändert, um diese auf die räumliche Verteilung und den Maßstab einer zweiten diagnostischen Untersuchung, die mit einem zweiten Diagnoseinstrument erhoben wird, abzustimmen;
Vergleichen der Funktionsausgabe für den Patienten mit einer Wahrscheinlichkeitsverteilung, die aus den Messungen bei normalen Subjekten erzeugt wurde, um eine Wahrscheinlichkeit der Normalität anzugeben; und
Anzeigen des Zustands der Funktionsausgabe im Verhältnis zum Normalzustand auf einer Grafikanzeige.

2. Verfahren nach Anspruch 1, ferner umfassend die Funktionsausgabe.

3. Verfahren nach Anspruch 1, wobei die Umwandlungsfunktion dafür konfiguriert ist, die Ähnlichkeit der Ergebnisse der beiden diagnostischen Untersuchungen über die Patientenpopulation zu maximieren.

4. Verfahren nach Anspruch 1, wobei die diagnostischen Untersuchungen eine Strukturuntersuchung und eine Funktionsuntersuchung des Auges beinhalten.

5. Verfahren nach Anspruch 1, wobei mindestens eine der diagnostischen Untersuchungen ausgewählt ist aus der Gruppe bestehend aus: Gesichtsfelduntersuchung, Analyse der retinalen Nervenfaserschicht, Analyse des Sehnervhomogenats, Analyse der Ganglienzellen und Dicke der inneren Netzhaut an der Makula.

6. Verfahren zur Analyse des Anomaliegrades von Netzhautgewebe im Auge eines Patienten, umfassend:
Erheben von zwei oder mehr Messungen des Auges des Patienten mithilfe zweier unterschiedlicher diagnostischer Untersuchungen mithilfe eines oder mehrerer Diagnoseinstrumente;
Anwenden einer Umwandlungsfunktion auf die Messungen aus einer der diagnostischen Untersuchungen, die die räumliche Verteilung und den Maßstab der Messungen verändert, um diese auf die räumliche Verteilung und den Maßstab der zweiten diagnostischen Untersuchung abzustimmen;
Kombinieren der umgewandelten Messungen aus der ersten diagnostischen Untersuchung und der Messungen aus der zweiten diagnostischen Untersuchung zur Erzeugung einer Ausgabe, die dafür optimiert ist, zwischen normal und krankhaft zu unterscheiden;
Vergleichen der kombinierten Ausgabe für den Patienten mit einer Wahrscheinlichkeitsverteilung, die aus den Messungen bei normalen Subjekten erzeugt wurde, um eine Wahrscheinlichkeit der Normalität anzugeben; und
Anzeigen des Zustands der Funktionsausgabe im Verhältnis zum Normalzustand auf einer Grafikanzeige.

7. Verfahren nach Anspruch 6, ferner umfassend Anzeigen der Funktionsausgabe.

8. Verfahren nach Anspruch 6, wobei die diagnostischen Untersuchungen eine Strukturuntersuchung und eine Funktionsuntersuchung des Auges beinhalten.

9. Verfahren nach Anspruch 6, wobei mindestens eine der diagnostischen Untersuchungen ausgewählt ist aus der Gruppe bestehend aus: Gesichtsfelduntersuchung, Analyse der retinalen Nervenfaserschicht, Analyse des Sehnervhomogenats, Analyse der Ganglienzellen und Dicke der inneren Netzhaut an der Makula.

10. Verfahren nach Anspruch 6, wobei ein oder mehrere Diagnoseinstrumente ausgewählt sind aus: Perimetrie, Scanning-Laser-Polarimetrie und optische Kohärenztomographie (OCT).

11. Verfahren nach Anspruch 10, wobei die zwei oder mehr Messungen mit demselben Diagnoseinstrument erfolgen.

12. Verfahren nach Anspruch 6, wobei die Funktionsausgabe in derselben Form erfolgt wie eine der Eingaben.

13. Verfahren nach Anspruch 6, wobei die Funktionseingaben nach der Zuverlässigkeit der einzelnen diagnostischen Untersuchungen gewichtet werden.

## Revendications

1. Procédé d'analyse du degré d'anomalie du tissu rétinien dans un oeil d'un patient comprenant les étapes suivantes :
collecter des mesures de la rétine de l'oeil du patient en utilisant un test de diagnostic issu d'un instrument de diagnostic ;
appliquer aux mesures une fonction de conversion qui modifie la distribution spatiale et l'échelle de mesure des mesures collectées pour les faire correspondre à la distribution spatiale et à l'échelle de mesure d'un deuxième test de diagnostic collecté avec un deuxième instrument de diagnostic ;
comparer la sortie fonctionnelle pour le patient à une distribution de probabilité créée à partir de mesures sur des sujets normaux pour indiquer une probabilité de normalité ; et
afficher l'état de la sortie fonctionnelle par rapport à la normale sur un écran graphique.

2. Procédé selon la revendication 1, comprenant en outre l'affichage de la sortie de la fonction.

3. Procédé selon la revendication 1, dans lequel la fonction de conversion est configurée pour maximiser la similitude des résultats des deux tests de diagnostic sur la population de patients.

4. Procédé selon la revendication 1, dans lequel les tests de diagnostic comportent un test structurel et un test fonctionnel de l'oeil.

5. Procédé selon la revendication 1, dans lequel au moins un des tests de diagnostic est choisi dans le groupe constitué par : un essai de champ visuel, une analyse de la RNFL, une analyse de l'ONH, une analyse des cellules ganglionnaires, et une épaisseur rétinienne à l'intérieur de la macula.

6. Procédé d'analyse du degré d'anomalie d'une rétine dans un oeil d'un patient comprenant les étapes suivantes :
collecter au moins deux mesures de l'oeil du patient en utilisant deux tests de diagnostic différents collectés en utilisant un ou plusieurs instruments de diagnostic ;
appliquer aux mesures issues d'un premier des tests de diagnostic une fonction de conversion qui modifie la distribution spatiale et l'échelle de mesure des mesures pour les faire correspondre à la distribution spatiale et à l'échelle de mesure du deuxième test de diagnostic ;
combiner les mesures converties issues du premier test de diagnostic et les mesures issues du deuxième test de diagnostic pour générer une sortie qui est optimisée pour établir une distinction entre la normale et une maladie ;
comparer la sortie combinée pour le patient à une distribution de probabilité créée à partir de mesures sur des sujets normaux pour indiquer une probabilité de normalité ; et
afficher l'état de la sortie fonctionnelle par rapport à la normale sur un écran graphique.

7. Procédé selon la revendication 6, comprenant en outre l'affichage de la sortie de la fonction.

8. Procédé selon la revendication 6, dans lequel les tests de diagnostic comportent un test structurel et un test fonctionnel de l'oeil.

9. Procédé selon la revendication 6, dans lequel au moins un des tests de diagnostic est choisi dans le groupe constitué par : un essai de champ visuel, une analyse de la RNFL, une analyse de l'ONH, une analyse des cellules ganglionnaires, et une épaisseur rétinienne à l'intérieur de la macula.

10. Procédé selon la revendication 6, dans lequel le ou les instruments de diagnostic sont choisis parmi : la périmétrie, la polarimétrie laser à balayage, et la tomographie de cohérence optique (OCT).

11. Procédé selon la revendication 10, dans lequel les au moins deux mesures sont prises en utilisant le même instrument de diagnostic.

12. Procédé selon la revendication 6, dans lequel la sortie de la fonction est sous la même forme qu'une des entrées.

13. Procédé selon la revendication 6, dans lequel les entrées de la fonction sont pondérées en fonction de la fiabilité des tests de diagnostic individuels.
